# EUROPÄISCHE PATENTSCHRIFT

(11) **EP 1 959 853 B1**
(45) Veröffentlichungstag und Bekanntmachung des Hinweises auf die Patenterteilung: **03.06.2009**
(21) Anmeldenummer: 06829095.6
(22) Anmeldetag: 22.11.2006
(51) Int. Cl.: A61B 18/02, A61B 18/00

(54) **KRYOCHIRURGISCHES GERÄT MIT EINER AUS DEM SOCKEL UND DEM STECKER VON KRYOSONDEN GEBILDETEN SONDENKUPPLUNG**
CRYOSURGICAL DEVICE WITH A PROBE COUPLING FORMED FROM THE SOCKET AND THE PLUG OF CRYOPROBES
APPAREIL CRYOCHIRURGICAL DOTE D'UN RACCORD DE SONDES FORME DU SOCLE ET DE LA FICHE DE CRYOSONDES

(30) Priorität: 16.12.2005 DE 102005060389; 25.01.2006 DE 102006003571
(43) Veröffentlichungstag der Anmeldung: 27.08.2008
(73) Patentinhaber: Erbe Elektromedizin GmbH, 72072 Tübingen (DE)
(72) Erfinder: GEISELHART, Franz, 72770 Reutlingen (DE)
(74) Vertreter: Bohnenberger, Johannes
(86) Internationale Anmeldenummer: PCT/EP2006/011199
(87) Internationale Veröffentlichungsnummer: WO 2007/073810

(56) Entgegenhaltungen:
- GB-A- 2 093 964
- GB-A- 2 289 413
- US-A- 4 146 030

## Beschreibung

Die Erfindung betrifft ein kryochirurgisches Gerät mit einer aus einem Sockel und einem Stecker von Kryosonden gebildeten Sondenkupplung nach dem Oberbegriff des Hauptanspruches. Solche Geräte finden in der Chirurgie überall dort Anwendung, wo sie besondere Vorteile zeigen oder Hochfrequenzchirurgie oder andere Verfahren einzusetzen sich verbietet. So kann man etwa Tumore bei deren ungünstiger Verteilung aus der Leber kaum herausschneiden, vielmehr wird hier krankhaft entartetes Gewebe mittels Tieffrieren abgetötet und im Übrigen im Körper belassen. Insbesondere mit flexiblen Sonden werden auch heute schon Fremdkörper aus Körperhöhlen durch Festfrieren an der Kryosonde extrahiert, so z.B. verschluckte und dabei versehentlich eingeatmete Erdnusskerne, die aus den Aternwegen entfernt werden müssen. Mechanische Greiftechniken verbieten sich hier, weil die Gefahr, den Erdnusskern zu zerbröseln, zu groß ist.

Um in der Chirurgie tiefzufrieren, gibt es verschiedene Verfahren. Eines stützt sich auf den Joule-Thomson-Effekt: die Atome bzw. Moleküle eines sich expandierenden Gases unterhalb der Inversionstemperatur arbeiten gegen die gegenseitige Anziehung an, so dass das Gas an innerer Energie verliert. Es kühlt ab. Dieser Effekt wird bei einer Reihe kryochirurgischer Methoden angewandt. Als expandierendes Gas- im Folgenden Arbeitsgas genannt - kommen üblicherweise CO₂ oder N₂O, das in der Anästhesie auch als Lachgas bekannt ist, in Frage, weil diese Gase in der Medizin wegen diverser Gründe verbreitet sind. Sie sind weder brennbar noch giftig, haben einen großen Joule-Thomson-Koeffizienten (µ) und sind unter Normaltemperatur verflüssigbar, so dass in der Druckgäsflasche über der flüssigen Phase stets eine Gasphase unter konstantem Druck vorgehalten werden kann.

Kryochirurgiegeräte der eben beschriebenen Art verfügen über ein. Reservoir, das ausreichend viel Arbeitsgas bereit hält, daneben über Sonden, die an die zu behandelnde Fläche im Körper verbracht werden, ferner über Zuleitungen, welche die Sonden durchsetzen und innerhalb der Sonden das Arbeitsgas in das Innenlumen der Sonden entlassen, wo es expandiert und in Folge dessen die Spitzen der Sonden abkühlt. Da die Sonden vorzugsweise aus thermisch leitfähigem Material gefertigt sind, ist ein Ableiten der Gewebewärme über die Sonden und damit eine Kühlwirkung gewährleistet.

Nachdem das tiefzufrierende Gewebe bzw. ein etwaiger Fremdkörper auf hinreichende tiefe Temperaturen abgekühlt ist, soll zu einem gegebenen Zeitpunkt ein Auftauen einsetzen, dabei aber am Gerät keine weiteren Vorrichtungen nötig macht, die das Auftauen überhaupt erst ermöglichen. Es bietet sich an, den Joule-Thomson-Effekt nun einfach umzukehren, indem das Gas unterhalb der Inversionstemperatur verdichtet wird. Hierfür müssen die Sonden an eine Entlüftung angeschlossen sein, die ihrerseits ein Ventil aufweist. Gesetzt den Fall, das Ventil fällt aus, aber die Sonde wird weiter mit Gas befüllt wird, so baut sich ein Druck auf, dem die Sonde standhalten muss. Dazu muss sie druckstabil ausgelegt sein. Soll keine Unfallgefahr bestehen, kommen deshalb für dieses Verfahren technisch bedingt derzeit nur starre Sonden in Betracht. Um die Sicherheit zu gewährleisten, wird der Gasweg gern provisorisch mit Schläuchen extern um das Rücklaufventil herumgeleitet und das Arbeitsgas so der Gasentsorgung zugeführt. Es kann zu Betriebsstörungen des Geräts kommen, wenn der externe Anschluss für diese Prozedur nicht verschlossen wird. Wird dabei das Arbeitsgas einfach in die Umgebungsluft des Operationssaals entlassen, kann insbesondere bei Lachgas leicht die zulässige Arbeitsplatzkonzentration (MAK) von 100 ppm überschritten werden.

Dokument GB2093964 offenbart ein Gerät nach dem Oberbegriff von Anspruch 1.

Der Erfindung liegt die Aufgabe zu Grunde, ein Kryochirurgiegerät der oben beschriebenen Art so weiter zu bilden, dass es die genannten Nachteile nicht mehr aufweist und unabhängig vom Kenntnisstand des Bedienpersonals sicher zu bedienen ist.

Diese Aufgabe wird durch eine Vorrichtung nach Patentanspruch 1 gelöst.

Ein wesentlicher Punkt der Erfindung liegt darin, dass verschiedene Kryosonden, seien sie starr oder flexibel, einfach und sicher und unabhängig vom Kenntnisstand des Bedienpersonals an das Kryogerät angeschlossen werden können. Dabei werden allein durch die Kupplungsgeometrie der entsprechende Rücklauf für starre Kryosonden (über eine Leitung mit einem Ventil) und der entsprechende Rücklauf für flexible Kryosonden (über eine Leitung ohne Ventil) in der Gasentsorgungsvorrichtung gewährleistet.

Bei einer ersten Ausführungsform der Erfindung sind die zu der Sondenkupplung gehörenden Leitüngszu- und Leitungsrückläufe durch den Sockel direkt mit einer Gasentsorgungsvorrichtung des Kryochirurgiegeräts verbunden. Dadurch wird erreicht, dass das Bedienpersonal beim Einsatz von flexiblen Sonden den Rücklauf zur Gasentsorgung des Kryochirurgiegerätes nicht mehr provisorisch mit meist nicht dafür vorgesehenen Schläuchen extern um das Rücklaufventil herumleiten muss und dadurch die durch "nicht verschließen" des Anschlusses möglichen Betriebsstörungen und mögliche Arbeitsplatz-konzentrationsüberschreitungen des Kühlgases vermieden werden.

Weiterhin haben die einsteckbaren Steckerbereiche der Kryosondenstecker einen runden Querschnitt und jeweils an den Öffnungen des Leitungszulaufes und Leitungsrücklaufes den Steckerbereich umlaufende Nuten. Die Nuten brauchen dazu nacht gesondert hergestellt zu werden. Durch den Abstand der Dichtungen bilden sich ringförmige Kammern, die geeignet sind, als Gaskanäle zu dienen. Dadurch wird erreicht, dass die Stecker auf einfache Weise vom Bedienpersonal in den Sockel eingesteckt werden können, wobei der Zulauf und der Rücklauf des Kühlgases zu den entsprechenden Kryosonden in jeder möglichen Stellung des eingesteckten Steckerbereiches gewährleistet wird und mögliche Fehler durch falsches Einstecken ausgeschlossen sind.

Außerdem sind die Nuten des Leitungsrücklaufs und des Leitungszulaufs des jeweiligen Steckerbereichs und eines korrespondierenden Sockelbereichs des Sockels durch Dichtungen, insbesondere durch O-Ringe, gasdicht voneinander getrennt, um die entsprechende Verbindung zwischen den Rückläufen und Zuläufen zwischen Sockel und Steckerbereich sicherzustellen, wobei der Leitungszulauf der ersten Kryosonde und der zweiten Kryosonde an der gleichen Stelle des Steckers und des Sockels angeordnet ist, so dass für die starre und flexible Sonde der Zulauf des Kühlgases durch die Zulaufleitung vom Reservoir des Kryochirurgiegerätes über den Sockelbereich in den Zulauf des entsprechenden Steckerbereichs gewährleistet ist. Zusätzlich führen der Leitungsrücklauf einer ersten starren Kryosonde über eine Leitung mit einem Ventil und der Leitungsrücklauf einer zweiten flexiblen Kryosonde über eine Leitung ohne Ventil zur Gasentsorgungsvorrichtung des Kryochirurgiegerätes. Dadurch wird erreicht, dass die starre Kryosonde unter Ausnutzung des umgekehrten Joule-Thomson-Effekts erwärmt werden kann, wobei das Gas durch das in der Leitung befindliche Ventil unterhalb der Inversionstemperatur verdichtet wird, und das Kühlgas in der flexiblen Sonde ohne Verdichtung durch ein Ventil, und somit ohne Unfallgefahr durch unvorhergesehenen Druckaufbau, in die Gasentsorgungsvorrichtung des Kryochirurgiegerätes abgeführt werden kann.

Des Weiteren ist es möglich, dass der Steckerbereich im Sockel des Kryochirurgiegerätes fixierbar ausgebildet ist. Dadurch wird sichergestellt, dass der Stecker nicht durch möglichen Druck von dem zu- und rücklaufenden Kühlgas aus dem Sockel herausgedrückt wird oder das Kühlgas des Zulaufs und/oder Rücklaufs in einen nicht vorgesehenen Zulauf und/oder Rücklauf gelangt. Der Verschluss kann dabei z.B; durch ein Außengewinde an dem Sockel und eine entsprechend angebrachte Mutter an dem Steckerbereich verwirklicht werden, oder aus einem lösbaren Klickverschluss oder durch eine Rastung entsprechend lösbaren Verschluss bestehen, oder auch durch ein am unteren Ende des Steckerbereichs.angebrachten Magneten und/oder eine am unteren Sockelbereich angebrachte Metallplatte oder Magneten gebildet werden. Eine Entlüftungsöffnung im hinteren Teil der Buchse ermöglicht ein leichteres Stecken, weil das Einstecken kein Luftpolster bilden kann. Ferner ist damit sicher gestellt, dass keine axialen Kräfte durch Druck den Stecker wieder herausdrücken. In diesem Fall kann eine Steckerfixierung auch entfallen.

In einer anderen Ausführungsform der Erfindung kann der Steckerbereich der ersten und der zweiten Kryosonde durch einen separaten Adapter gebildet werden, wobei der erste und zweite Steckerbereich an eine erste bzw. zweite herkömmliche Kryosoride angepasst sind. Dadurch wird erreicht, dass auch ältere Modelle verschiedener Kryosonden mit dem Kryochirurgiegerät der vorliegenden Erfindung verwendet werden können. Des Weiteren kann der Sockelbereich auch durch einen separaten Adapter gebildet werden, wobei der separate Adapter über Leitungen an die,Steuerung des Kryochirurgiegerätes und/oder das Reservoir für Kühlgas und/oder das Ventil und/oder die Gasentsorgungsvorrichtung für das rücklaufende Kühlgas angeschlossen ist. Dadurch wird erreicht, dass auch ältere Modelle des Kryochirurgiegerätes für den Einsatz der Kryosonden der vorliegenden Erfindung und/oder für den Einsatz von älteren Kryosonden mit entsprechenden Adaptern eingesetzt werden können.

Eine weitere Ausführungsform der Erfindung sieht vor, dass die Sondenkupplung in einem Kryochirurgiegerät mit einer Vorrichtung zur Kontrolle der Eisballgestaltung enthalten ist, wobei die Eisballgestaltung durch ein den Eisball umgebendes elektromagnetisches Feld kontrolliert wird. Dadurch wird erreicht, dass starre und flexible Sonden mit für die Erzeugung des elektromagnetischen Feldes notwendigen elektrischen Bereichen einfach und sicher in das entsprechende Kryochirurgiegerät eingesteckt werden können.

Weitere Ausführungsformen der Erfindung ergeben sich aus den Unteransprüchen.

Nachfolgend wird die Erfindung anhand eines Ausführungsbeispiels näher beschrieben, das anhand der Abbildungen näher erläutert werden soll. Hierbei zeigen
- Figur 1: eine perspektivische Darstellung eines Kryochirurgiegerätes mit einer starren Sonde und einer flexiblen Sonde
- Figur 2a: eine perspektivische Darstellung eines Steckerbereiches einer starren Kryosonde
- Figur 2b: eine Schnittdarstellung entlang der Linie A-B in Fig. 1 eines in einem Sockel eingesteckten Steckerbereiches einer starren Kryosonde
- Figur 3a: eine perspektivische Darstellung eines Steckerbereiches einer flexiblen Kryosonde
- Figur 3b: eine Schnittdarstellung entlang der Linie A-B in Fig. 1 eines in einen Sockel eingesteckten Steckerbereichs einer flexiblen Kryosonde
- Figur 4a: eine perspektivische Darstellung eines Adapters für den Steckerbereich einer herkömmlichen starren Sonde
- Figur 4b: eine Schnittdarstellung eines Adapters für den Steckerbereich einer herkömmlichen starren Sonde mit eingestecktem Stecker
- Figur 5a: eine perspektivische Darstellung eines Adapters für den Steckerbereich einer herkömmlichen flexiblen Sonde
- Figur 5b: eine Schnittdarstellung eines Adapters für den Steckerbereich einer herkömmlichen flexiblen Sonde mit eingestecktem Stecker
- Figur 6a: eine perspektivische Darstellung eines Adapters für den Sockelbereich eines herkömmlichen Kryochirurgiegerätes mit einem Anschlussstutzen für eine das Ventil umgehende Rücklaufleitung zur Gasentsorgung
- Figur 6b: eine Schnittdarstellung eines Adapters für den Sockelbereich eines herkömmlichen Kryochirurgiegerätes mit eingesteckter starren Sonde, und
- Figur 6c: eine perspektivische Darstellung eines Adapters für den Sockelbereich eines herkömmlichen Kryochirurgiegerätes mit eingesteckter flexiblen Sonde;

In der nachfolgenden Beschreibung werden für gleiche und gleich wirkende Teile dieselben Bezugsziffern verwendet.

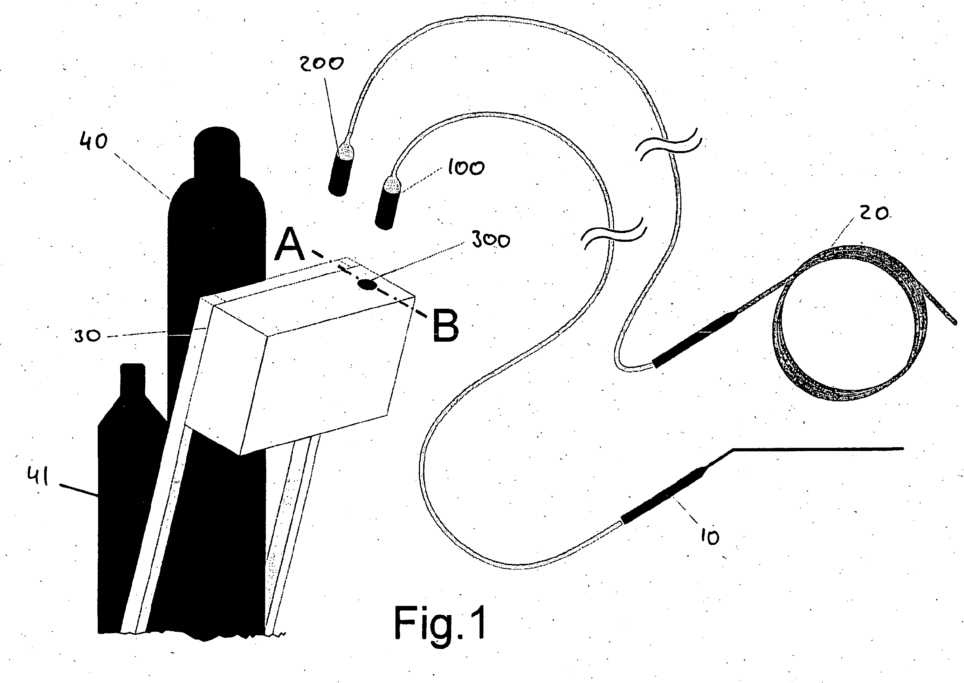

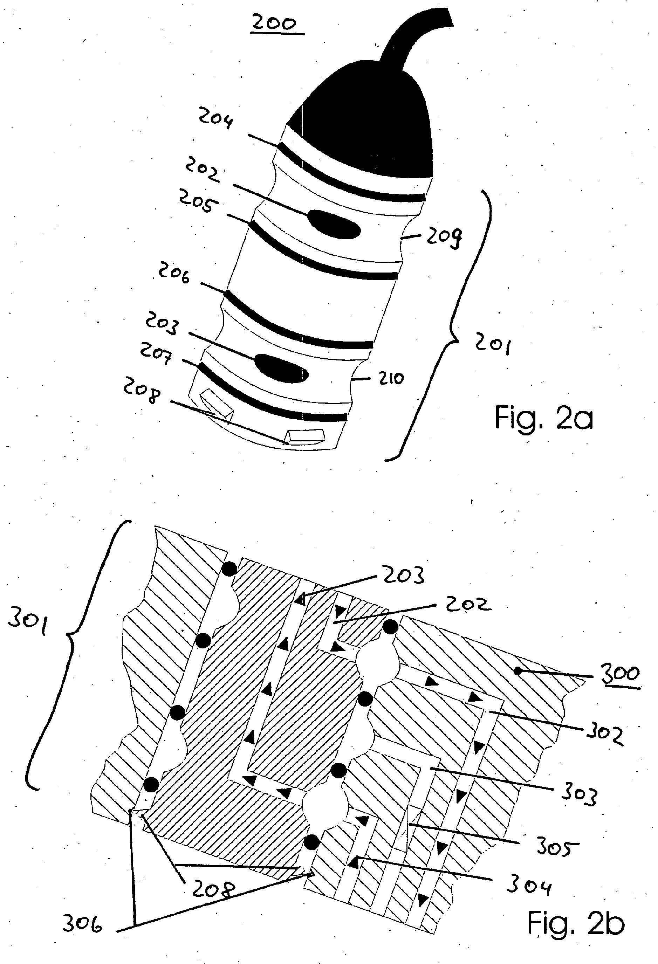

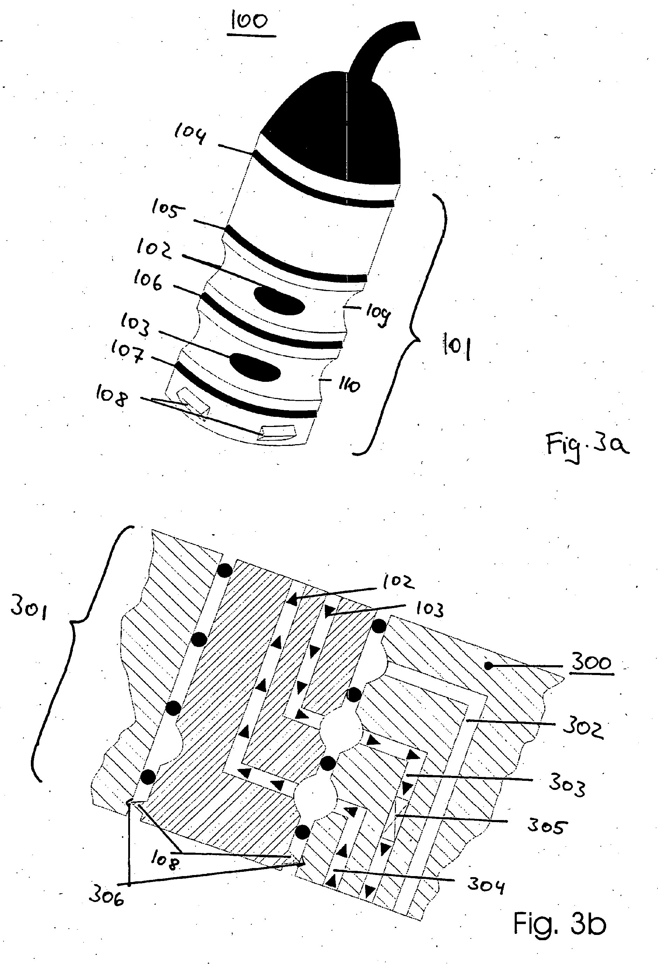

In dem in Figur 1 dargestellten Ausführungsbeispiel ist ein Kryochirurgiegerät mit einer Steuerung 30, einem Reservoir 40, einem Behälter für die Gasentsorgung 41, einer starren ersten Sonden 10 und einer flexiblen zweiten Sonde 20 dargestellt. Die Steuerung 30 des Kryochirurgiegerätes umfasst einen Sockel 300 zum Anschluss der starren ersten Sonde 10 oder zum Anschluss der flexiblen zweiten Sonde 20, wobei ein Stecker 100 der starren ersten Kryosonde 10 oder ein Stecker 200 der flexiblen zweiten Kryosonde 20 und der Sockel 300 des Kryochirurgiegerätes eine Sondenkupplung bilden. Das Reservoir 40 des Kryochirurgiegerätes ist mit einem Kühlgas für die Kühlung der entsprechenden Kryosonde 10, 20 gefüllt und über Leitungen an die Steuerung 30 und einen Leitungszulauf 304 (siehe Fig. 2b) des Sockels 300 verbunden. Die Gasentsorgungsvorrichtung 41 ist mit einem jeweiligen Leitungsrücklauf 102, 202 (siehe Fig. 2b, 3b) der Kryosonden 10, 20 über beide Leitungsrückläufe 302, 303 des Sockels 300, einmal mit einem Ventil 305 und einmal ohne einem Ventil, verbunden.

Figur 2a zeigt eine perspektivische Darstellung des Steckers 200 der flexiblen zweiten Kryosonde 20. Ein Steckerbereich 201 hat zwei kreisförmige Öffnungen 202, 203, wobei die Öffnung 202 am oberen Ende des Steckerbereiches 201 und die Öffnung 203 am unteren Ende des Steckerbereiches 201 angeordnet ist. Auf der jeweiligen Höhe der Öffnungen 202, 203 befindet sich eine den Steckerbereich 201 umlaufende Nut 209, 210, die mindestens die Breite der jeweiligen Öffnung 202, 203 aufweist. Auf beiden Seiten der jeweiligen Öffnungen 202, 203 und Nuten 209, 210 sind entsprechende den Steckerbereich 201 umlaufende Dichtungen 204, 205, 206, 207 so angeordnet, dass sie im eingesteckten Zustand des Steckers 200 eine gasdichte Abtrennung zwischen dem Leitungszulauf 304, 203 und dem Leitungsrücklauf 302, 202 gewährleisten. In dem in Figur 2a dargestellten Stecker 200 befindet sich am unteren Ende des Steckerbereiches 201 eine durch eine Rastung lösbare Fixierung 208, die beim Einstecken in die am Sockelbereich 201 korrespondierend angeordneten Kerben 306 einschnappt.

In Verbindung mit der in Figur 2b dargestellten Schnittzeichnung des in einem Sockelbereich 301 eingesteckten Steckerbereiches 201 sollen der Aufbau der vorliegenden Sondenkupplung und die Wirkung zwischen dem Stecker 200 und dem Sockel 300 des Kryochirurgiegerätes näher erläutert werden.

In der Steuerung 30 des Kryochirurgiegerätes führen die Zulaufleitung 304 und die zwei Rücklaufleitungen 302, 303 zu dem Sockelbereich 301 des Sockels 300, wobei in dem Ausführungsbeispiel der Figur 2b der Leitungsrücklauf 302 des Sockels 300 ohne Ventil mit dem Leitungsrücklauf 202 der flexiblen zweiten Kryosonde 20 verbunden ist, und der Leitungszulauf 304 mit dem Leitungszulauf 203 der flexiblen zweiten Kryosonde 20 verbunden ist. Dadurch wird gewährleistet, dass das Kühlgas ohne entsprechenden Druckaufbau durch ein Ventil von der flexiblen zweiten Kryosonde 20 in die Gasentsorgungsvorrichtung 41 abgeleitet wird und somit eine potentielle Schädigung der flexiblen zweiten Kryosonde 20 vermieden wird. Beim Einstecken des Steckers 200 den flexiblen zweiten Kryosonde 20 in den Sockel 300 des Kryochirurgiegerätes rastet die Fixierung 208 in die korrespondierenden Kerben 306 des Sockels 300 ein und sorgt somit für einen sicheren Halt des Steckers 200 im Sockel 300. Durch das Einstecken des Steckers 200 wird der Leitungszulauf 304 der Steuerung 30 über den Sockel 304. automatisch mit dem Leitungszulauf 203 der flexiblen zweiten Kryosonde 20 verbunden. Des Weiteren wird der zur Gasentsorgungsvorrichtung 41 führende Leitungsrücklaüf 302 der Steuerung 30 mit dem Leitungsrücklauf 202 der flexiblen zweiten Kryosonde 20 verbunden, wobei der Leitungsrücklauf 302 der Steuerung 30 am Ventil 305 vorbei und somit direkt in die Gasentsorgungsvorrichtung 41 führt. Die die Öffnungen 202, 203 umlaufenden Nuten 209, 210 sorgen dafür, dass der Zu- und Rücklauf des Kühlgases in jeder möglichen Stellung des eingesteckten Steckers 200 gewährleistet ist.

Figur 3a zeigt eine perspektivische Darstellung eines Steckers 100 einer starren ersten Kryosonde 10. Ein Steckerbereich 101 hat zwei kreisförmige Öffnungen 102, 103, wobei die Öffnung 102 im mittleren Bereich des Steckerbereiches 101 zwischen Dichtungen 105 und 106 angeordnet ist, und eine Öffnung 103 am unteren Ende des Steckerbereiches 101 zwischen den Dichtungen 106 und 107 angeordnet ist. Auf der jeweiligen Höhe der Öffnungen 102, 103 befindet sich eine den Steckerbereich 101 umlaufende Nut 109, 110 die mindestens die Breite der jeweiligen Öffnung 102, 103 aufweist. Die Dichtungen 104, 105, 106 und 107 sind so angeordnet, dass sie im eingesteckten Zustand des Steckers 100 eine gasdichte Abtrennung zwischen dem Leitungszulauf 304, 103 und dem Leitungsrücklauf 302, 102 gewährleisten. In dem in Figur 3a dargestellten Stecker 100 befindet sich am unteren Ende des Steckerbereiches 101 eine durch eine Rastung lösbare Fixierung 108, die beim Einstecken in die am Sockelbereich 301 korrespondierend angeordneten Kerben 306 einschnappt.

In Verbindung mit der in Figur 3b dargestellten Schnittzeichnung des in den Sockelbereich 301 eingesteckten Steckerbereiches 101 soll der Aufbau der vorliegenden Sondenkupplung und die Wirkung zwischen dem Stecker 100 und dem Sockel 300 des Kryochirurgiegerätes näher erläutert werden.

In der Steuerung 30 des Kryochirurgiegerätes führen eine Zulaufleitung 304 und die zwei Rücklaufleitungen 302, 303 zu dem Sockelbereich 301 des Sockels 300, wobei in dem Ausführungsbeispiel der Figur 3b der Leitungsrücklauf 303 des Sockels 300 mit Ventil 305 mit dem Leitungsrücklauf 102 der starren ersten Kryosonde 10 verbunden ist, und der Leitungszulauf 304 mit dem Leitungszulauf 103 der starren ersten Kryosonde 10 verbunden ist. Dadurch wird erreicht, dass beim Abführen des Kühlgases durch das Ventil 305 in die Gasentsorgungsvorrichtung 41 das Kühlgas unterhalb der Inversionstemperatur verdichtet wird und die starre erste Kryosonde durch Umkehrung des Joule-Thomson-Effekts schneller auftaut. Beim Einstecken des Steckers 100 der starren ersten Kryosonden 10 in den Sockel 300 des Kryochirurgiegerätes rastet die Fixierung 108 in die korrespondierenden Kerben 306 des Sockels 300 ein und sorgt somit für einen sicheren Halt des Steckers 100 im Sockel 300. Beim Einstecken des Steckers 100 wird der Leitungszulauf 304 der Steuerung 30 über den Sockel 304 automatisch mit dem Leitungszulauf 103 der starren ersten Kryosonde 10 verbunden und der zur Gasentsorgungsvorrichtung 41 führende Leitungsrücklauf 303 der Steuerung 30 wird automatisch mit dem Leitungsrücklauf 102 der starren ersten Kryosonde 10 verbunden, wobei der Leitungsrücklauf 303 der Steuerung 30 durch das Ventil 305 in die Gasentsorgungsvorrichtung 41 führt. Die die Öffnungen 102, 103 umlaufenden Nuten 109, 110 sorgen dafür, dass der Zu- und Rücklauf des Kühlgases in jeder möglichen Stellung des eingesteckten Steckers 100 gewährleistet ist.

Die Fixierung des Steckers 100, 200 im Sockel 300 kann auch durch andere für die Stecker 100, 200 der Kryosonden 10, 20 geeignete Fixierungen realisiert werden. So kann der Stecker 100, 200 z.B. auch durch ein außen am Sockel 300 angebrachtes Außengewinde und eine am oberen Steckerbereich beweglich angebrachte und zu dem Außengewinde korrespondierende Mutter im Sockel 300 befestigt werden. Außerdem kann die Fixierung des Steckers 100, 200 im Sockel 300 auch durch einen Klickverschluss erfolgen, oder durch ein am unteren Ende des Steckerbereichs 101 angebrachten Magneten und/oder eine am unteren Sockelbereich 101 angebrachte Metallplatte oder Magneten gebildet werden. Weitere dem Fachmann bekannte oder offensichtliche Fixierungsmöglichkeiten für einen Stecker 100 in einem Sockel 300 sollen hiermit auch einbezogen sein.

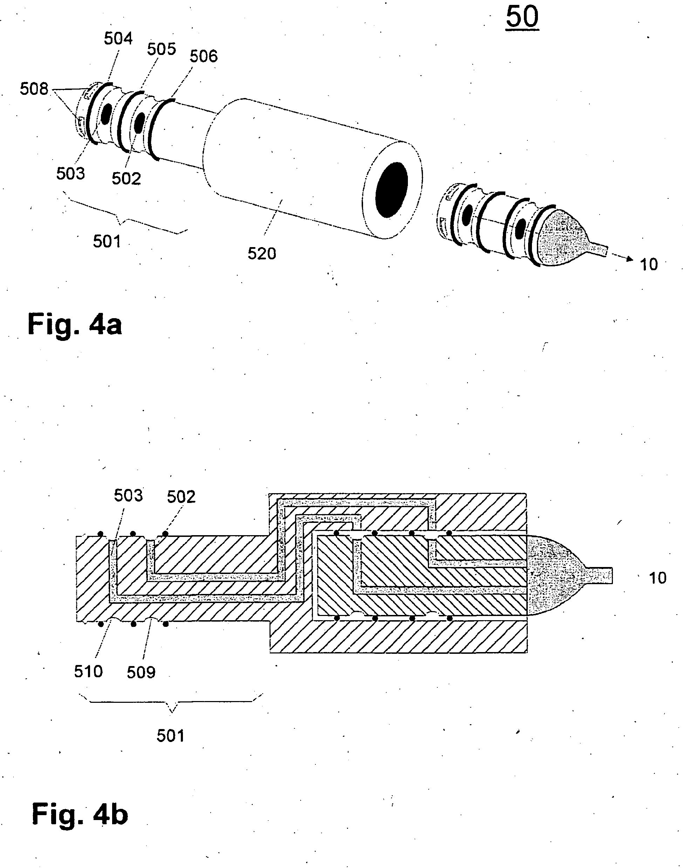

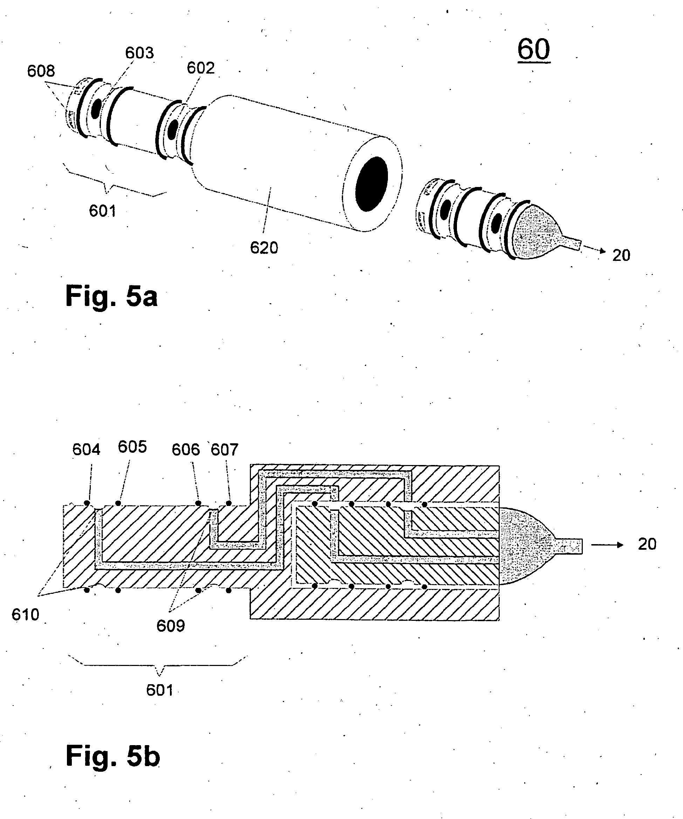

Ein weiteres Ausführungsbeispiel einer Sondekupplung ist in Fig. 4a, 4b, 5a, und 5b dargestellt. Hierbei handelt es sich um zwei Adapter 50, 60 für die Stecker von starren und flexiblen Kryosonden älterer und für den Einsatz mit dem Kryochirurgiegerät nicht geeignete Modelle. Der Adapter besteht aus einem für den Einsatz in das Kryochirurgiegerät geeigneten Steckerbereich 501, 601 und einem mit dem jeweiligen Stecker der herkömmlichen Modelle kompatiblen Sockel 520, 620. Der Steckerbereich 501, 601 des Adapters 50, 60 umfasst jeweils zwei Öffnungen für die Zu- und Rücklaufleitungen 502, 503, 602, 603 und umfasst auf der jeweiligen Höhe der Öffnungen 502, 503, 602, 603 den Steckerbereich umlaufende Nuten 509, 510, 609, 610. Weiterhin sind die Öffnungen 502, 503, 602, 603 und die Nuten 509, 510, 609, 610 durch Dichtungen 504 bis 506 und 604 bis 607, insbesondere O-Ringe, im eingesteckten Zustand gasdicht voneinander getrennt. Am unteren Ende des Steckerbereiches 501, 601 des Adapters 50, 60 befindet sich z.B. eine für die Fixierung des Adapters 50, 60 in den Sockelbereich 301 des Kryochirurgiegerätes geeignete Rastung 508, 608. Die Öffnungen der Zu- und Rücklaufleitungen 502, 503, 602, 603 des Adapters 50, 60 sind so angeordnet, dass sie beim Einstecken in den Sockel 300 des Kryochirurgiegerätes der vorliegenden Erfindung jeweils mit der zu der entsprechenden flexiblen oder starren Kryosonde 10, 20 geeigneten Zulaufleitung 304 und Rücklaufleitung 302, 303 verbunden werden. Dabei wird die Zulaufleitung der herkömmlichen starren Kryosonde über den Adapter 50 mit der Zulaufleitung 304 der Steuerung des Kryochirurgiegerätes verbunden, und die Rücklaufleitung der herkömmlichen starren Kryosonde über den Adapter 50 mit der Rücklaufleitung 303 mit Ventil 305 der Steuerung des Kryochirurgiegerätes verbunden. Die Zulaufleitung einer herkömmlichen flexiblen Kryosonde wird beim Einstecken in den Sockel 300 des Kryochirurgiegerätes der vorliegenden Erfindung über den Adapter 60 mit der Zulaufleitung 304 und die Rücklaufleitung der herkömmlichen flexiblen Kryosonde wird über den Adapter 60 mit der das Ventil 305 umgehenden Rücklaufleitung 302 verbunden.

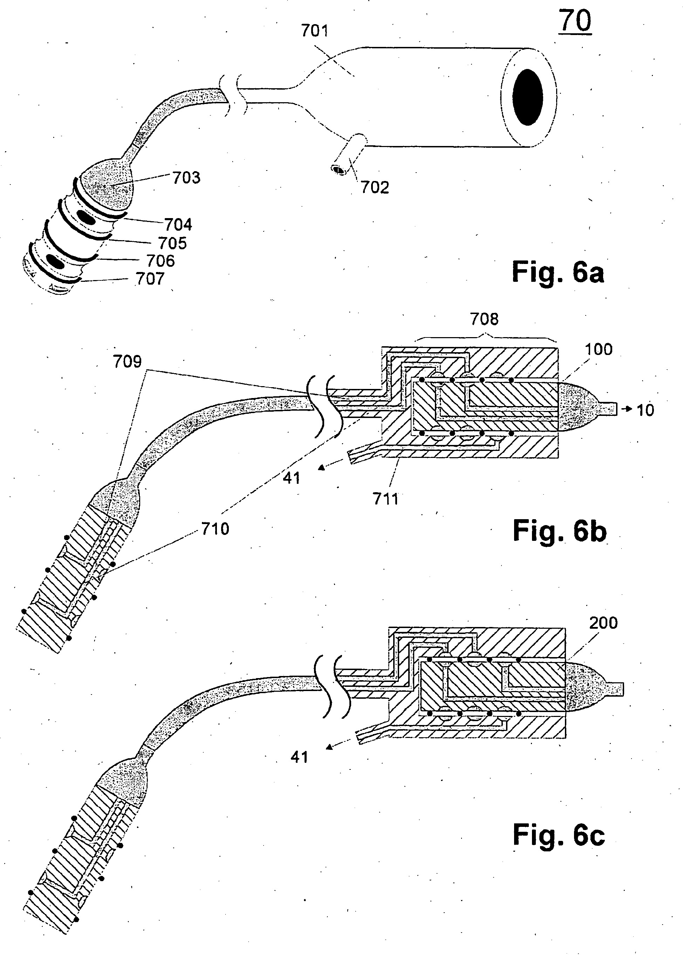

In einem anderen Ausführungsbeispiel einer Sondenkupplung ist in den Figuren 6a, 6b und 6c ein zum Anschluss der flexiblen und starren Kryosonden 10, 20 geeigneter Adapter 70 für ältere Modelle eines Kryochirurgiegerätes dargestellt. Der Adapter umfasst einen Sockel 701, einen daran verbundener zum Anschluss an ein herkömmliches Kryogerät geeigneter Stecker 703 und einen aus dem unteren äußeren Teil des Sockels herausstehender Stutzen 702. Ein innerer Sockelbereich 708 umfasst drei Öffnungen von Leitungen 709, 710, und 711 die so angeordnet sind, dass beim Einstecken einer flexiblen Kryosonde 20 eines Kryochirurgiegerätes der hier vorliegenden Erfindung die Zulaufleitung 203 der flexiblen Kryosonde 20 über die Zulaufleitung 710 des Adapters 70 mit der Zulaufleitung des herkömmlichen Kryochirurgiegerätes verbunden ist, und die Rücklaufleitung der flexiblen Kryosonde 20 über die Rücklaufleitung 711 des Adapters 70 und über eine externe Leitung (in Fig. 6a, 6b, 6c nicht dargestellt) ohne Ventil mit der Gasentsorgungsvorrichtung des herkömmlichen Kryochirurgiegerätes verbunden ist, und, dass beim Einstecken einer starren Kryosonde 10 eines Kryochirurgiegerätes der hier vorliegenden Erfindung die Zulaufleitung 103 der starren Kryosonde 10 über die Zulaufleitung 710 des Adapters 70 mit der Zulaufleitung des herkömmlichen Kryochirurgiegerätes verbunden ist, und die Rücklaufleitung 102 der starren Kryosonde 10 über die Rücklaufleitung 709 des Adapters 70 mit der ein Ventil enthaltenen Rücklaufleitung des Kryochirurgiegerätes verbunden ist. Weiterhin sind die Öffnungen der Leitungen 709, 710, 711 durch Dichtungen 704 bis 707, insbesondere O-Ringe, im eingesteckten Zustand gasdicht voneinander getrennt.

In einem weiteren Ausführungsbeispiel können die Adapter 50, 60, 70 auch für eine Verbindung zwischen einem herkömmlichen Kryochirurgiegerät und einer herkömmlichen starren oder flexiblen Kryosonde genutzt werden. Dabei wird der Adapter 70 in den Sockel des herkömmlichen Kryochirurgiegeräts gesteckt und die Adapter 50, 60 auf die jeweiligen Stecker der herkömmlichen flexiblen und/oder starren Kryosonden aufgesteckt, womit die jeweils geeignete Verbindung zwischen Zulauf- und Rücklaufleitungen der flexiblen und/oder starren Kryosonde gewährleistet ist.

Die Sondenkupplung kann auch an einem Kryochirurgiegerät das eine Vorrichtung zur Kontrolle der Gestaltung der Eisballbildung durch entsprechend erzeugte elektromagnetische Felder umfasst, intern eingebaut sein oder durch entsprechende Adapter an dem Kryochirurgiegerät oder an den Steckern der Kryosonden realisiert werden. Möglich sind auch Steckerkombinationen mit Kontakten zu Temperatursensoren oder elektrischen Auftauhilfen.

### Bezugszeichenliste

- 10: erste, starre Kryosonde
- 20: zweite, flexible Kryosonde
- 30: Steuerung für Kryochirurgiegerät
- 40: Reservoir für Kühlgas
- 41: Gasentsorgungsvorrichtung
- 50: Adapter für Stecker einer starren Kryosonde
- 60: Adapter für Stecker einer flexiblen Kryosonde
- 70: Adapter für Sockel eines Kryochirurgiegerätes
- 100: erster Kryosondenstecker
- 101: Steckerbereich erste Kryosonde
- 102: Leitungsrücklauf erste Kryosonde
- 103: Leitungszulauf erste Kryosonde
- 104: Dichtung erste Kryosonde
- 105: Dichtung erste Kryosonde
- 106: Dichtung erste Kryosonde
- 107: Dichtung erste Kryosonde
- 108: Fixierung erste Kryosonde
- 109: Nut am Leitungsrücklauf
- 110: Nut am Leitungszulauf
- 200: zweiter Kryosondenstecker
- 201: Steckerbereich zweite Kryosonde
- 202: Leitungsrücklauf zweite Kryosonde
- 203: Leitungszulauf zweite Kryosonde
- 204: Dichtung zweite Kryosonden
- 205: Dichtung zweite Kryosonde
- 206: Dichtung zweite Kryosonden
- 207: Dichtung zweite Kryosonde
- 208: Fixierung zweite Kryosonde
- 209: Nut am Leitungsrücklauf
- 210: Nut am Leitungszulauf
- 300: Sockel
- 301: Sockelbereich
- 302: Leitungsrücklauf für zweite Kryosonde
- 303: Leitungsrücklauf für erste Kryosonde
- 304: Leitungszulauf für Kryosonden
- 305: Ventil
- 306: Kerbe für Fixierungen 108, 208
- 501: Steckerbereich Adapter für starre Sonde
- 502: Leitungsrücklauf Adapter für starre Sonde
- 503: Leitungszulauf Adapter für starre Sonde
- 504: Dichtung für Adapter 50
- 505: Dichtung für Adapter 50
- 506: Dichtung für Adapter 50
- 508: Fixierung (Rastung)
- 509: Nut am Leitungsrücklauf
- 510: Nut am Leitungszulauf
- 601: Steckerbereich Adapter für flexible Sonde
- 602: Leitungsrücklaüf Adapter für flexible Sonde
- 603: Leitungszulauf Adapter für flexible Sonde
- 604: Dichtung für Adapter 60
- 605: Dichtung für Adapter 60
- 606: Dichtung für Adapter 60
- 607: Dichtung für Adapter 60
- 609: Nut am Leitungsrücklauf
- 610: Nut am Leitungszulauf
- 701: Sockel für Adapter 70
- 702: Stutzen für Leitungsrücklauf flexible Sonde
- 703: herkömmlicher Stecker
- 704: Dichtung für Adapter 70
- 705: Dichtung für Adapter 70
- 706: Dichtung für Adapter 70
- 707: Dichtung für Adapter 70
- 708: innerer Sockelbereich des Adapters 70
- 709: Rücklaufleitung für starre Sonde
- 710: Leitungszulauf
- 711: Leitungsrücklauf flexible Sonde

## Patentansprüche

1. Kryochirurgisches Gerät, mit einer Steuerung (30) zum Zuführen und/oder Abführen mindestens eines Kühlgases zu einer Kryosonde (10, 20) über einen Rücklauf beziehungsweise einen Zulauf und mit mindestens einem Sockel (300) zum Anschluss von mindestens einer ersten. Kryosonde (10) und einer zweiten Kryosonde (20), welche in ihrem Leitungsrücklauf einen anderen Vor- und/oder Rücklaufdruck als die erste Kryosonde (10) benötigt, über Stecker (100, 200) an den Kryosönden (10, 20), wobei
der Sockel (300) mindestens zwei Leitungsrückläufe (7, 8) und/oder Leitungszuläufe (302, 303) aufweist **dadurch gekennzeichnet, dass** der Sockel (300) und die Stecker (100, 200) Sondenkupplungen bilden, und dass die erste Kryosonde (10) einen Stecker (100) aufweist, der sich vom Stecker (200) der zweiten Kryosonde (20) derart unterscheidet, dass das Abführen und/oder Zuführen des Kühlgases aus/zu der zweiten Kryosonde (20) beim Einstecken in den Sockel (300) über andere Leitungsrückläufe (202, 302) und/oder Leitungszuläufe (203, 304) erfolgt, als das Abführen beziehungsweise Zuführen des Kühlgases aus/zu der ersten Kryosonde (10) beim Einstecken in diesen Sockel (300).

2. Kryochirurgisches Gerät nach Anspruch 1,
**dadurch gekennzeichnet, dass**
die zu der Sondenkupplung gehörenden Leitungszu- und Leitungsrückläufe (302, 303, 304) des Sockels (300) direkt mit einem Reservoir (40) und einer Gasentsorgungsvorrichtung (41) des Kryochirurgiegerätes verbunden sind.

3. Kryochirurgisches Gerät nach einem der vorhergehenden Ansprüche,
**dadurch gekennzeichnet, dass**
in den Sockel (300) einsteckbare Steckerbereiche (101, 201) der Stecker (100, 200) mit rundem, Querschnitt jeweils an Öffnungen des Leitungszulaufs 103, 203) und Leitungsrückläufs (102, 202) den Steckerbereich (101, 201) umlaufende Nuten (109, 110, 209, 210) aufweisen, die den Zulauf und den Rücklauf des Kühlgases zu der entsprechenden Kryosonde (10, 20) in jeder möglichen Stellung des eingesteckten Steckerbereichs (101, 201) gewährleistet.

4. Kryochirurgisches Gerät nach einem der vorhergehenden Ansprüche, insbesondere nach Anspruch 3,
**dadurch gekennzeichnet, dass**
die Nuten (109, 110, 209, 210) des Leitungsrücklaufs (102, 202) und des Leitungszulaufs (103, 203) des jeweiligen Steckerbereichs (101, 201) und eines korrespondierenden Sockelbereichs (301) des Sockels (300) durch Dichtungen (104, 105, 106, 107, 204, 205, 206, 207), insbesondere durch O-Ringe, gasdicht voneinander getrennt sind.

5. Kryochirurgisches Gerät nach einem der vorhergehenden Ansprüche, insbesondere nach Anspruch 4,
**dadurch gekennzeichnet, dass**
der Leitungszulauf (103, 203) bei der ersten Kryosonde (10) und der zweiten Kryosonde (20) an der gleichen Stelle des Steckers (100, 200) und des Sockels (300) angeordnet ist.

6. Kryochirurgisches Gerät nach einem der vorhergehenden Ansprüche, insbesondere nach Anspruch 5,
**dadurch gekennzeichnet, dass**
der Leitungsrücklauf (102) der ersten Kryosonde (100) ein Rücklauf des Kühlgases über eine Leitung (303) mit einem Ventil (305) zur Gasentsorgung, und der Leitungsrücklauf (202) der zweiten Kryosonde (20) ein Rücklauf des Kühlgases über eine Leitung (302) ohne ein Ventil zur Gasentsorgung ist.

7. Kryochirurgiegerät nach einem der vorhergehenden Ansprüche,
**dadurch gekennzeichnet, dass**
der Steckerbereich (101, 201) im Sockel (300) fixierbar ausgebildet ist.

8. Kryochirurgiegerät nach Anspruch 5,
**dadurch gekennzeichnet, dass**
die Fixierung (108, 208) im Sockel (300) eine lösbare Klick-Fixierung, oder eine durch eine Rastung lösbare Fixierung ist, oder durch ein am unteren Ende des Steckerbereichs angebrachten Magneten und/oder eine am unteren Sockelbereich angebrachte Metallplatte oder Magneten gebildet ist.

9. Kryochirurgisches Gerät nach einem der vorhergehenden Ansprüche,
**dadurch gekennzeichnet, dass**
der Steckerbereich (101, 201) der ersten und der zweiten Kryosonde (10, 20) durch einen separaten Adapter (50, 51) gebildet ist, und
der erste und zweite Steckerbereich (101, 201) an die erste (10) beziehungsweise zweite Sonde (20) angepasst ist.

10. Kryochirurgisches Gerät nach einem der vorhergehenden Ansprüche,
**dadurch gekennzeichnet, dass**
der Sockelbereich (301) durch einen separaten Adapter (60) gebildet ist, und
der separate Adapter (60) über Leitungen mit der Steuerung (30) des Kryochirurgiegeräts und/oder einem Reservoir (40) für Kühlgas und/oder einem Reduzierventil (305) verbunden ist.

## Claims

1. Cryosurgical device having a control (30) for supplying and/or removing at least one cooling gas to a cryoprobe (10, 20) via a return or a feed and having at least one socket (300) for connection of at least one first cryoprobe (10) and one second cryoprobe (20), which requires a different advance pressure and/or return pressure than the first cryoprobe (10) in its pipe return, via plugs (100, 200) to the cryoprobes (10,20), wherein the socket (300) has at least two pipe returns and/or pipe feeds (302, 303), **characterised in that** the socket (300) and the plugs (100, 200) form probe couplings, and **in that** the first cryoprobe (10) has a plug (100) which differs from the plug (200) of the second cryoprobe (20) such that the removal and/or supply of the cooling gas from/to the second cryoprobe (20) is effected via further pipe returns (202, 302) and/or pipe feeds (203, 304) during insertion into the socket (300) rather than the removal or supply of the cooling gas from/to the first cryoprobe (10) during insertion into this socket (300).

2. Cryosurgical device according to claim 1, **characterised in that** the pipe feeds and pipe returns (302, 303, 304) of the socket (300) belonging to the probe coupling are connected directly to a reservoir (40) and a gas-disposal device (41) of the cryosurgical device.

3. Cryosurgical device according to one of the preceding claims, **characterised in that** plug regions (101, 201), which can be inserted in the socket (300), of the plugs (100, 200) having round cross-section have grooves (109, 110, 209, 210) surrounding the plug region (101, 201) in each case at openings of the pipe feed (103, 203) and pipe return (102, 202), which guarantees the feed and the return of the cooling gas to the corresponding cryoprobe (10, 20) in every possible position of the inserted plug region (101, 201).

4. Cryosurgical device according to one of the preceding claims, in particular according to claim 3, **characterised in that** the grooves (109, 110, 209, 210) of the pipe return (102, 202) and of the pipe feed (103, 203) of the particular plug region (101, 201) and of a corresponding socket region (301) of the socket (300) are separated from one another in gastight manner by seals (104, 105, 106, 107, 204, 205, 206, 207), in particular by O rings.

5. Cryosurgical device according to one of the preceding claims, in particular according to claim 4, **characterised in that** the pipe feed (103, 203) for the first cryoprobe (10) and the second cryoprobe (20) is arranged at the same point of the plug (100, 200) and of the socket (300).

6. Cryosurgical device according to one of the preceding claims, in particular according to claim 5, **characterised in that** the pipe return (102) of the first cryoprobe (100) is a return of the cooling gas via a pipe (303) having a valve (305) for gas disposal and the pipe return (202) of the second cryoprobe (20) is a return of the cooling gas via a pipe (302) without a valve for gas disposal.

7. Cryosurgical device according to one of the preceding claims, **characterised in that** the plug region (101, 201) is designed to be fixable in the socket (300).

8. Cryosurgical device according to claim 5, **characterised in that** the fixing (108, 208) in the socket (300) is a releasable click fixing, or a fixing which can be released by a catch, or is formed by a magnet attached to the lower end of the plug region and/or a metal plate or magnet attached to the lower socket region.

9. Cryosurgical device according to one of the preceding claims, **characterised in that** the plug region (101, 201) of the first and the second cryoprobe (10, 20) is formed by a separate adapter (50, 51), and the first and second plug region (101, 201) is adapted to the first (10) or second probe (20).

10. Cryosurgical device according to one of the preceding claims, **characterised in that** the socket region (301) is formed by a separate adapter (60), and the separate adapter (60) is connected via pipes to the control (30) of the cryosurgical device and/or a reservoir (40) for cooling gas and/or a reducing valve (305).

## Revendications

1. Appareil cryochirurgical doté d'un dispositif de commande (30) pour l'amenée et/ou l'évacuation d'au moins un gaz réfrigérant vers/depuis une cryosonde (10, 20) via un circuit de retour ou un circuit d'amenée et d'au moins un socle (300) pour le raccordement d'au moins une première cryosonde (10) et une deuxième cryosonde (20) qui nécessite dans son conduit de retour une pression d'arrivée et/ou de retour différente de celle de la première sonde (10), via des fiches (100, 200) prévues sur les cryosondes (10, 20), le socle (300) présentant au moins deux conduits de retour (302, 303) et/ou conduits d'amenée,
**caractérisé en ce que**
le socle (300) et les fiches (100, 200) forment des accouplements de sonde et **en ce que** la première cryosonde (10) présente une fiche (100) qui se différencie de la fiche (200) de la deuxième cryosonde (20) par le fait que l'évacuation et/ou l'amenée du gaz réfrigérant hors de/vers la deuxième cryosonde (20), quand celle-ci est enfichée dans le socle (300), se fait via d'autres conduits de retour (202, 302) et/ou conduits d'amenée (203, 304) que l'évacuation ou l'amenée du gaz réfrigérant hors de/vers la première cryosonde (10) quand celle-ci est enfichée dans le socle (300).

2. Appareil cryochirurgical selon la revendication 1,
**caractérisé en ce que**
les conduits d'amenée et de retour (302, 303, 304) du socle (300) faisant partie de l'accouplement de sonde sont directement reliés à un réservoir (40) et à un dispositif d'élimination du gaz (41) de l'appareil cryochirurgical.

3. Appareil cryochirurgical selon l'une quelconque des revendications précédentes,
**caractérisé en ce que**
les zones d'enfichage (101, 201) des fiches (100, 200) desection transversale ronde et aptes à être enfichées dans le socle (300) présentent, au niveau d'orifices du conduit d'amenée (103, 203) et du conduit de retour (102, 202), des gorges (109, 110, 209, 210) faisant le tour de la zone d'enfichage (101, 201) qui garantissent l'amenée et le retour du gaz réfrigérant vers/depuis la sonde correspondante (10, 20) quelle que soit la position dans laquelle la zone d'enfichage (101, 201) est enfichée.

4. Appareil cryochirurgical selon l'une quelconque des revendications précédentes, notamment selon la revendication 3,
**caractérisé en ce que**
les gorges (109, 110, 209, 210) du conduit de retour (102, 202) et du conduit d'amenée (103, 203) des zones d'enfichage (101, 201) et d'une zone correspondante (301) du socle (300) sont séparées les unes des autres de manière étanche aux gaz au moyen de dispositifs d'étanchéité (104, 105, 106, 107, 204, 205, 206, 207), notamment au moyen de joints toriques.

5. Appareil cryochirurgical selon l'une quelconque des revendications précédentes, notamment selon la revendication 4,
**caractérisé en ce que**
les conduits d'amenée (103, 203) de la première cryosonde (10) et de la deuxième cryosonde (20) sont disposés au même endroit de la fiche (100, 200) et du socle (300).

6. Appareil cryochirurgical selon l'une quelconque des revendications précédentes, notamment selon la revendication 5,
**caractérisé en ce que**
le conduit de retour (102) de la première cryosonde (10) assure le retour du gaz réfrigérant vers le dispositif d'élimination du gaz via un conduit (303) doté d'une soupape (305), et le conduit de retour (202) de la deuxième cryosonde (20) assure le retour du gaz réfrigérant vers le dispositif d'élimination du gaz via un conduit (302) sans soupape.

7. Appareil cryochirurgical selon l'une quelconque des revendications précédentes,
**caractérisé en ce que**
la zone d'enfichage (101, 201) est conçue de manière à pouvoir être immobilisée dans le socle (300).

8. Appareil cryochirurgical selon la revendication 5,
**caractérisé en ce que**
le moyen d'immobilisation (108, 208) dans le socle (300) est une fixation réversible par enclipsage ou encliquetage, ou est formé par un aimant rapporté sur l'extrémité inférieure de la zone d'enfichage et/ou par une plaque métallique ou un aimant rapporté(e) sur la zone inférieure du socle.

9. Appareil cryochirurgical selon l'une quelconque des revendications précédentes,
**caractérisé en ce que**
la zone d'enfichage (101, 201) de la première et de la deuxième cryosondes (10, 20) est formée par un adaptateur séparé (50, 51) et **en ce que**
la première et la deuxième zones d'enfichage (101, 201) sont adaptées respectivement à la première sonde (10) et à la deuxième sonde (20).

10. Appareil cryochirurgical selon l'une quelconque des revendications précédentes,
**caractérisé en ce que**
la zone de socle (301) est formée par un adaptateur séparé (60) et **en ce que** l'adaptateur séparé (60) est relié au dispositif de commande (30) de l'appareil cryochirurgical et/ou à un réservoir (40) de gaz réfrigérant et/ou à une soupape de réduction de pression (305) via des conduits.
